(19) Europäisches Patentamt
European Patent Office
Office européen des brevets

(11) **EP 1 792 582 B1**

(12) **EUROPEAN PATENT SPECIFICATION**

(45) Date of publication and mention
of the grant of the patent:
**04.04.2018 Bulletin 2018/14**

(51) Int Cl.:
*A61M 29/02* (2006.01)          *A61L 31/14* (2006.01)
*A61L 31/10* (2006.01)          *A61L 31/16* (2006.01)

(21) Application number: **05781550.8**

(22) Date of filing: **01.09.2005**

(86) International application number:
**PCT/JP2005/016002**

(87) International publication number:
**WO 2006/027994 (16.03.2006 Gazette 2006/11)**

(54) **INDWELLING STENT**

DAUERSTENT

ENDOPROTHESE VASCULAIRE A DEMEURE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **08.09.2004 JP 2004260893**

(43) Date of publication of application:
**06.06.2007 Bulletin 2007/23**

(73) Proprietor: **Kaneka Corporation
Osaka (JP)**

(72) Inventors:
• **NISHIDE, Takuji,**
**Osaka Plant, KANEKA CORPORATION**
**Settsu-shi, Osaka 5660072 (JP)**
• **TAKIGUCHI, Yuki,**
**Osaka Plant, KANEKA CORPORATION**
**Settsu-shi, Osaka 5660072 (JP)**
• **NAKANO, Ryoji,**
**Osaka Plant, KANEKA CORPORATION**
**Settsu-shi, Osaka 5660072 (JP)**
• **FUKAYA, Kohei,**
**Osaka Plant, KANEKA CORPORATION**
**Settsu-shi, Osaka 5660072 (JP)**
• **KAWATSU, Masaji,**
**Takasago Plant, KANEKA CORP.**
**Takasago-shi, Hyogo 6760027 (JP)**

(74) Representative: **Gille Hrabal
Brucknerstrasse 20
40593 Düsseldorf (DE)**

(56) References cited:
WO-A1-00/32255          WO-A1-01/87372
WO-A1-2004/026361          JP-A- 6 218 063
JP-A- 8 089 585          JP-A- 10 052 502
JP-A- 2004 000 565          JP-A- 2004 097 810
JP-A- 2004 222 953          JP-A- 2004 523 275
US-A- 5 824 048

## Description

TECHNICAL FIELD

The present invention relates to a medical stent for placement in body for use in dilating blood vessel stenosis.

BACKGROUND ART

One of the serious problems on health we face currently is blood vessel stenosis caused by arteriosclerosis. In particular, stenosis of cardiac coronary artery is known to lead to severe diseases such as angina pectoris and myocardial infarction, very frequently resulting in death. One of the methods for treatment of such a blood vessel stenosis site, which is widely practiced as a minimal invasive treatment, is angioplasty (PTA, PTCA) of dilating the stenosis site by expansion of a small balloon inserted into blood vessel. However, the angioplasty leads to repeated stenosis (restenosis) at high probability. Various treatments such as atrectomy, laser treatment, and radiation treatment were studied for reducing the frequency of restenosis (restenosis rate), and recently, a method of placing a stent is used more widely.

The stent is a medical device that is placed in a blood vessel or other lumen in the body for preservation of the lumen size, after dilation of the corresponding stenosed or occluded site when it is stenosed or occluded. The stent is generally made of a metal, a polymer, or the composite thereof, and stents of a metal such as stainless steel are used most commonly.

In treatment with a stent, the stent is inserted into blood vessel with a catheter and expanded for mechanical support of the vascular lumen when it becomes in contact with the unhealthy region of vascular wall. Although the restenosis rate after treatment by such a stent placement method becomes statistically significantly smaller than that by angioplasty only with a balloon, it is still significantly high currently. For example in the case of cardiac coronary artery, the restenosis rate after stent placement therapy is reported to be as high as approximately 20 to 30%. The restenosis is said to be caused by excessive reaction for restoring the blood vessel physically damaged by stent placement, i.e. , rapid neointimal hyperplasia, for example, by growth of smooth muscle cells in media after blood vessel damage, migration of the grown smooth muscle cells into intima, and migration of T cells and macrophages into the intima.

Recently for reduction of the restenosis rate after stent placement, proposed is a method of coating an antiocclusion drug on the stent. In Patent Document 1, drugs such as anticoagulant, antiplatelet, antibacterial, antitumor, antimicrobial, anti-inflammatory, antimetabolic, and immunosuppressive agents are studied as the antiocclusion drug. As for the immunosuppressive agent, cyclosporine, tacrolimus (FK506), sirolimus (rapamycin), mycophenolate mofetil, and the analogs thereof (everolimus, ABT-578, CCI-779, AP23573, etc.) are studied for reduction of the restenosis rate as they are coated on stent. For example, Patent Document 2 discloses a stent coated with an immunosuppressive agent sirolimus (rapamycin), while Patent Document 3 discloses a stent coated with an antitumor drug taxol (paclitaxel). Alternatively, Patent Documents 4 and 5 disclose a stent coated with tacrolimus (FK506).

Tacrolimus (FK506), compound having a CAS number of 104987-11-3, is disclosed, for example, in Patent Document 6. Tacrolimus (FK506), which is considered to inhibit mainly production of differentiation-growth factors, i.e., cytokines such as IL-2 and INF-$\gamma$, in T cell by forming a complex with FK506-binding protein (FKBP) in the cell, is well known to be used as a preventive or treatment drug for prevention of rejection during organ transplantation and also for autoimmune diseases. Nonpatent Literature 1 confirms that tacrolimus (FK506) has an action to inhibit growth of human vascular cell.

As for the method of applying a medicine on stent, Patent Document 1 discloses use of a polymer, favorably a biodegradable polymer, as a carrier for the medicine. Patent Document 7 discloses use of a biodegradable polymer, and an example of the biodegradable polymer is polylactic acid.

In coating a medicine on stent with a polymer, troubles such as the exfoliation and cracking coating layer associated with stent expansion need to be prevented. The exfoliation and cracking of the coating layer, which frequently leads to severe disorders such as occlusion of blood vessel by excessive thrombus generation in the acute period after stent placement, are extremely dangerous. There is no description on typical methods of preventing such exfoliation and cracking in Patent Documents 1 and 7. US Patent Number 5,824,048 discloses a method for delivering a therapeutic substance to a body lumen utilizing an intravascular stent having a coating comprising a polymer and a therapeutic substance in a solid/solid solution with the polymer. On the other hand, when a medicine is applied on stent by using a polymer, the medicine should be held on the stent in an amount sufficient for expression of the therapeutic effect. One of the easiest ways to increase the amount of medicine retained is to raise the ratio of the medicine to the polymer.

During application of the medicine, the polymer has two grossly divided roles: a role as a binder controlling adhesion of the medicine onto the stent surface and a role as a reservoir controlling release of the medicine into blood stream and organs . Increase of the ratio of medicine to polymer generally results in deterioration of the functions as a binder and also as a reservoir. Thus, increase of the ratio of medicine to polymer results in increase of the possibility of exfoliation and cracking of the coating layer associated with stent expansion and completion of medicinal elution in a shortened period of time, because of deterioration in slow-release property of the stent.

The slow-drug-release property is quite important, when the medicinal virtues are desirably retained over an extended period of time. As described above, the restenosis is seemingly caused by excessive reaction for restoring blood vessel physically damaged by stent placement, i.e., rapid neointimal hyperplasia, for example, by growth of smooth muscle cells in media after blood vessel damage, migration of the grown smooth muscle cells into intima, and migration of T cells and macrophages into the intima. Such biological reactions, i.e., causes of restenosis, start immediately after stent placement and become largest approximately three to six months after stent placement. Accordingly, it is preferable to release the medicine consistently over an extended period of time after stent placement for reduction of the restenosis rate with the medicine on the stent surface. There are many disclosed methods for controlled delivery of medicine, from these viewpoints.

Patent Document 7 discloses a stent having a composite layer containing a biological activator and a polymer substance formed thereon and a barrier layer formed on the composite layer, wherein the barrier layer is formed by low-energy plasma polymerization of a monomer gas. The document discloses that presence of the barrier layer is effective in controlling release of the biological activator, but the method, in which the barrier layer is formed by low-energy plasma polymerization, has problems that it demands an additional special facility, cannot use a non-volatile monomer species for the barrier layer, and thus, is still unsatisfactory from the point of flexibility in use.

Alternatively, Patent Document 8 discloses a stent having an undercoat layer of a hydrophobic elastomer material containing a biological active substance, and a topcoat layer covering at least part of the undercoat layer and practically containing no eluting material. The document shows that the topcoat controls delivery of the biologically active substance. However, the controllability of drug delivery by the method is relatively lower, and thus, the stent is yet to have a controlled-delivery efficiency sufficient for reducing the restenosis rate after stent placement.

Patent Document 1: Japanese Unexamined Patent Publication No. 5-502179
Patent Document 2 : Japanese Unexamined Patent Publication No. 6-009390
Patent Document 3 : Japanese Unexamined Patent Publication No. 9-503488
Patent Document 4: WO 02/065947
Patent Document 5: EP 1254674
Patent Document 6 : Japanese Unexamined Patent Publication No. 61-148181
Patent Document 7 : Japanese Unexamined Patent Publication No. 2002-531183
Patent Document 8 : Japanese unexamined patent publication 2004-754
Nonpatent Literature 1: Paul J. Mohacsi MD, et al. The Journal of Heart and Lung Transplantation, May 1997, Vol. 16, No. 5, 484-491

DISCLOSURE OF THE INVENTION

Problems to be Solved by the Invention

An object of the present invention, which was made under the circumstances above, is to provide a stent carrying a medicine for prevention of restenosis by using a polymer as its binder and reservoir that allows increase of the amount of the medicine retained on the stent and controlled release of the medicine as much as possible without the cracking and exfoliation of the coating layer associated with stent expansion.

Means to Solve the Problems

After intensive studies to solve the problems above, the inventors have invented a tube-shaped stent expandable toward outside in the radial direction of the tube expanding, including a stent main body containing a material non-degradable in the body as its base stent material, a coating layer containing a medicine and a polymer formed on at least part of the stent main body surface, wherein the coating layer consists of an internal layer and an external layer, the medicine/polymer weight ratio by weight in the internal layer is higher than that in the external layer and the medicine is contained in an effective amount in the external layer wherein the medicine/polymer weight ratio in the internal layer is 0.50 or more and 1.60 or less and the medicine/polymer weight ratio in the external layer is 0.10 or more and 0.40 or less. The present invention also relates to a stent for placement in body having the coating layer over the almost entire surface of the external, internal, and side faces of the stent main body.

The present invention also relates to the stent for placement in body wherein the polymer is a biodegradable polymer.

The present invention also relates to the stent for placement in body wherein the biodegradable polymer is polylactic acid, polyglycolic acid, or a lactic acid-glycolic acid copolymer.

The present invention also relates to the stent for placement in body wherein the biodegradation period of the polymer for the internal layer is almost same or longer than that of the polymer for the external layer.

In the invention, the medicine contained in the internal layer is preferably the same as the medicine contained in the external layer, and more preferably, the medicine is an immunosuppressive agent. The immunosuppressive agent is preferably tacrolimus (FK506), cyclosporine, sirolimus (rapamycin), azathioprine, mycophenolate mofetil more preferably tacrolimus (FK506).

The medicine contained in the internal layer may be different from the medicine contained in the external layer, and preferably, the medicine contained in the internal layer is an immunosuppressive agent and the medicine contained in the external layer is an anti-inflammatory agent. the immunosuppressive agent is preferably tacrolimus (FK506), cyclosporine, sirolimus (rapamycin), azathioprine, mycophenolate mofetil, and the antiinflammatory agent is preferably dexamethasone, hydroxycortisone, cortisone, desoxycorticosterone, fludrocortisone, betamethasone, prednisolone, prednisone, methyl prednisolone, paramethasone, triamcinolone, flumethasone, fluocinolone, fluocinonide, fluprednisolone, halcinonide, flurandrenolide, meprednisone, medrysone, cortisol, 6a-methylprednisolone, triamcinolone, betamethasone, salicylic acid derivative, diclofenac, naproxen, sulindac, indomethacin, and particularly preferably, the immunosuppressive agent is tacrolimus (FK506) and the anti-inflammatory agent is dexamethasone.

In the invention, the medicine/polymer weight ratio in the internal layer is 0.50 or more and 1.60 or less.

Also in the invention, the medicine/polymer weight ratio in the external layer is 0.10 or more and 0.40 or less. In the invention, the medicine/polymer weight ratio in the internal layer is 0,50 or more and 1.60 or less and the medicine/polymer weight ratio in the external layer is 0.10 or more and 0.40 or less. Advantageous Effects of the Invention

The stent for placement in body according to the invention has a stent main body containing a material non-degradable in the body as its base stent material, a coating layer containing a medicine and a polymer as principal components formed on at least part of the stent main body surface, wherein the coating layer consists of an internal layer and an external layer, the medicine/polymer weight ratio by weight in the internal layer is higher than that in the external layer, and the medicine is contained in an effective amount in the external layer, and thus , allows increase of the amount of the medicine retained on the stent and controlled release of the medicine as much as possible without the cracking and exfoliation of the coating layer associated with stent expansion. In addition, the stent for placement in body according to the invention can be prepared more easily than conventional stents for placement in body.

BRIEF DESCRIPTION OF THE DRAWINGS

Figure 1 is a development view of a stent.
Figure 2 is a schematic view of the stent.
Figure 3 is a SEM observation image of the stent obtained in Example 1.
Figure 4 is a SEM observation image of the stent obtained in Comparative Example 1.
Figure 5 is a graph showing the results of an in-vitro drug elution test.

BEST MODE OF CARRYING OUT THE INVENTION

The present invention relates to a tube-shaped stent comprising a stent main body that is expandable toward outside in the radial direction of the tube and contains a material non-degradable in the body as the base stent material, and a coating layer containing a medicine and a polymer as principal components on at least part of the stent main body surface, wherein the coating layer consists of an internal layer and an external layer, the medicine/polymer weight ratio by weight in the internal layer is higher than that in the external layer, and the medicine is contained in an effective amount in the external layer, wherein the medicine/polymer weight ratio in the internal layer is 0.50 or more and 1.60 or less and the medicine/polymer weight ratio in the external layer is 0.10 or more and 0.40 or less. The base stent material according to the present invention means a material for stent having no coating layer. The stent main body can be prepared by cutting a base stent material, such as a tube-shaped material, into the stent shape, for example by laser cutting. The coating layer is preferably formed on almost entire surface of the external, internal, and side faces of the stent main body. The stent main body having the coating on the almost entire surface is thus resistant to deposition of platelet on the surface of the stent placed in a body lumen, in particular in blood vessel, possibly preventing the occlusion of blood vessel by generation of an excessive amount of thrombus .

The "material non-degradable in the body" for use in the present invention is a material not easily degradable biologically; thus, it is not a material that no decomposition occurs at all in the body, but a material that can keep its original shape relatively for an extended period of time; and such a material is also included in the "material non-degradable in the body" according to the invention.

Examples of the materials non-degradable in the body according to the present invention, i.e. , base stent materials, include inorganic materials such as stainless steel, Ni-Ti alloys , Cu-Al-Mn alloys, tantalum, Co-Cralloys, iridium, iridium oxide, niobium, ceramics, and hydroxyapatite. The stent main body can be prepared by a method normally practiced by those who are skilled in the art, and, for example, it can be prepared by cutting a tube-shaped material tube of the base stent material into the stent shape for example by laser cutting, as described above. The stent may be polished electrically after the laser cutting. The material non-degradable in the body according to the present invention is not limited to an inorganic material, and a polymeric material such as polyolefin, polyolefin elastomer, polyamide, polyamide elastomer, polyurethane, polyurethane elastomer, polyester, polyester elastomer, polyimide, polyamide-imide, or polyether ether ketone may be used. The method of producing a stent main body by using such a polymeric material does not restenose the advantageous effects of the present invention, and any processing method may be used arbitrarily according to the material used. The stent according to present invention, which contains a material non-degradable in the body as its stent base material, retains its favorable stent strength for a longer period of time and is extremely more effective in vasodilating the stenosed or occluded site of blood vessel than a stent having its stent main body made of a biodegradable material.

When the coating layer has a single-layered structure, increase of the medicine/polymer weight ratio leads to increase in the amount of the medicine retained, but also causes a problem of more frequent cracking and exfoliation of the coating layer by stent expansion. On the other hand, decrease of the medicine/polymer weight ratio leads to reduction of the cracking and exfoliation of the coating layer caused by stent expansion, but also causes a problem of a low retention rate of the medicine. Thus when the coating layer has a single-layered structure, it is difficult to increase the amount of the medicine retained and control the cracking and exfoliation of the coating layer by stent expansion at the same time.

The present invention, which is aimed at solving the problem above, has a coating layer containing a medicine and a polymer as principal components formed on at least part of the stent surface, wherein the coating layer consists of an internal layer and an external layer, the medicine/polymer weight ratio by weight in the internal layer is higher than that in the external layer, and the medicine is contained in an effective amount in the external layer. Presence of the external layer having a lower medicine/polymer weight ratio leads to effective reduction of the cracking and exfoliation of the coating layer by stent expansion. Because the external layer has a medicine/polymer weight ratio lower than that of the internal layer, the medicine is eluted gradually in the early phase after stent placement. Because elution of the medicine in the internal layer starts after elution of the medicine in the external layer, the polymer contained in the external layer functions as a barrier layer and allows gradual release of the medicine from the internal layer having a relatively higher medicine/polymer weight ratio . In addition, because the internal layer has a high medicine/polymer weight ratio, the stent as a whole has a greater medicine content.

The ratio (by weight) of the internal layer to the external layer is decided arbitrarily according to the specification for the desirable stent for placement in the body. For example, the weight of the internal layer is preferably made larger than that of the external layer on a stent in the specification mainly aimed at increasing the medicine retention rate, while the weight of the external layer is preferably made greater than that of the internal layer on a stent in the specification mainly aimed at controlling delivery of the medicine.

A layer other than the internal and external layers may be formed additionally for improvement in medicine retention rate, control of medicine delivery, prevention of cracking and exfoliation of the coating layer by stent expansion, or the like. For example, an intermediate layer may be formed between the internal layer and the stent surface for prevention of cracking and exfoliation of the coating layer by stent expansion. The intermediate layer preferably contains only a polymer, more preferably a polymer having a weight-average molecular weight higher than those of the polymers contained in the internal and external layers.

The polymer contained in the internal or external layer is preferably a biodegradable polymer, more preferably polylactic acid, polyglycolic acid, or a lactic acid-glycolic acid copolymer. Polylactic acids are available in three kinds of structures depending on the optical activity of the lactic acid monomer: poly-L-lactic acid, poly-D-lactic acid, and poly-D, L-lactic acid, but polylactic acid in any structure shows the advantageous effects of the present invention. Use of a biodegradable polymer results in disappearance of all polymer by biodegradation in the chronic phase after stent placement and also in residual only of the stent base material in the body. It is possible to provide a stent higher in stability and reliability also in the chronic phase, easily by using a reliable metal material, such as SUS316L, as the stent base material.

The biodegradable polymers exemplified above have a glass transition temperature not lower than the body temperature, although it may vary according to the composition and the polymer weight, and thus, are in the rigid glass state at around body temperature. In addition, poly-L-lactic acid, poly-D-lactic acid, polyglycolic acid, and the like are known to show high crystallinity. For that reason, the biodegradable polymers exemplified above show a tensile strength higher and a tensile breaking elongation shorter than other polymers such as thermoplastic elastomers. Thus, use of such a biodegradable polymer in forming a coating layer on the surface of the stent main body, caused a problem that there was an extremely high possibility of cracking and exfoliation of the coating layer associated with stent expansion. However, it is possible to reduce the possibility of the cracking and exfoliation of the coating layer of the coating layer and to coat the biodegradable polymer and medicine exemplified above favorably, by forming the coating layer according to the invention.

When both the polymers for the internal and external layers are biodegradable polymers, the biodegradation period of the polymer for the internal layer is preferably almost same or longer than that of the polymer for the external layer. Unfavorably when the biodegradation period of the polymer for the internal layer is shorter than that of the polymer for the external layer, the internal layer disappears before the external layer, likely causing cracking and exfoliation of the external layer.

The biodegradation period of a biodegradable polymer is calculated by using the change in weight, strength, molecular weight, and the like of the biodegradable polymer as an indicator. Generally, the biodegradation period calculated from the molecular weight change is shortest, that calculated from the strength change is second shortest, and that calculated from the weight change is longest. The biodegradation period, independent of the indicator used for calculation, does not restenose the advantageous effects of the present invention. However, as described above, the biodegradation periods of a single biodegradable polymer calculated from different indicators are different from each other, and thus, the biodegradation period of the biodegradable polymer for the internal layer and that of the biodegradable polymer for the external layer should be the values calculated from the same indicator.

The method of forming the internal and external layers on the stent main body surface is not particularly limited. In a favorable method, a medicine and a polymer for the internal layer is dissolved in a solvent; the medicine and the polymer in the solution state is applied on the surface of the stent main body, and the solvent is removed: a medicine and a polymer for the external layer is dissolved in a solvent; the medicine and the polymer in the solution state applied on the surface of the internal layer and the solvent is removed. Alternatively, a film of medicine and polymer for the internal layer may be prepared separately and bonded to the stent main body, and a film of a medicine and the polymer for the external layer bonded to the surface of the internal layer. Yet alternatively, a medicine and a polymer for the internal layer may be dissolved in a solvent; the medicine and the polymer in the solution state is applied on the surface of the stent main body, and the solvent removed; and then, a film of medicine and polymer for the external layer be prepared separately and bonded to the surface of the internal layer, and yet alternatively, a film of medicine and polymer for the internal layer may be prepared separately and bonded to the stent main body, a medicine and a polymer for the external layer dissolved in a solvent and the medicine and the polymer in the solution state applied on the surface of the internal layer and the solvent removed. The method of forming the internal and external layers does not restenose the advantageous effects of the present invention, and various methods are used favorably.

When the polymer for the internal or external layer and a medicine are applied as they are dissolved in a solvent, the application method does not restenose the advantageous effects of the present invention. Thus, various methods, including the method of dipping the stent main body in each solution and the method of applying each solution on the stent main body by spraying, may be used. The solvent for use is not particularly limited. A solvent having a desirable solubility is favorably used, and two or more solvents may be used as a mixed solvent, for adjustment of volatility and others. The solute concentration is also not particularly limited, and the optimal concentration is determined, taking into consideration the surface smoothness or the like of the internal layer and the external layer. For adjustment of the surface smoothness, an excessive amount of the solution may be removed during the process of dissolving the polymer for the internal layer and a medicine in a solvent and applying the polymer in the solution state or/and after application thereof, or alternatively, during the process of dissolving the polymer for the external layer and a medicine in a solvent and applying the polymer in the solution state or/and after application thereof. The solvent-removing means include vibration, rotation, evacuation, and the like, and these means may be used in combination.

The medicine contained in the internal layer may be the same as or different from that contained in the external layer. When the medicine contained in the internal layer is the same as that in the external layer, the medicine is preferably an immunosuppressive agent, more preferably tacrolimus (FK506), cyclosporine, sirolimus (rapamycin), azathioprine, mycophenolate mofetil, and particularly preferably tacrolimus (FK506).

When the medicine contained in the internal layer is different from that contained in the external layer, the medicine contained in the internal layer is preferably an immunosuppressive agent and that in the external layer an anti-inflammatory agent; preferably, the immunosuppressive agent is tacrolimus (FK506), cyclosporine, sirolimus (rapamycin), azathioprine, mycophenolate mofetil, and the antiinflammatory agent is dexamethasone, hydroxycortisone, cortisone, desoxycorticosterone, fludrocortisone, betamethasone, prednisolone, prednisone, methyl prednisolone, paramethasone, triamcinolone, flumethasone, fluocinolone, fluocinonide, fluprednisolone, halcinonide, flurandrenolide, meprednisone, medrysone, cortisol,6a-methylprednisolone, triamcinolone, betamethasone, a salicylic acid derivative, diclofenac, naproxen, sulindac, indomethacin, and particularly preferably, the immunosuppressive agent is tacrolimus (FK506), and the anti-inflammatory agent dexamethasone.

Dexamethasone is an adrenocortical steroid, a compound having a CAS number of 50-02-2, having anti-inflammatory and antiallergic actions, influencing on metabolisms of sugar, protein, lipid, and others, and having applications such as chronic rheumatoid arthritis, bronchial asthma, atopic dermatitis, and others. When the medicine contained in the internal layer is tacrolimus (FK506) and that in the external layer dexamethasone, dexamethasone is mainly eluted from the external layer in the acute and subacute periods after stent placement, reducing the inflammatory reaction associated with stent placement by its anti-inflammatory action. Tacrolimus is mainly eluted from the internal layer in the subacute to chronic phase, preventing neointimal hyperplasia newly formed by overgrowth of the smooth muscle cells, i.e., organ-restoring reaction after stent placement.

The ratio of medicine/polymer by weight in the internal layer is 0.50 or more and 1.60 or less. A ratio of less than 0.50 is unfavorable, because it is difficult to raise the medicine retention rate efficiently. On the other hand, a ratio of more than 1.60 is also unfavorable, because it leads to more frequent cracking and exfoliation of the internal and external layers associated with stent expansion.

The medicine/polymer weight ratio in the external layer is 0.10 or more and 0.40 or less. A ratio of less than 0.10 is unfavorable, because the medicine retention rate in the external layer becomes smaller and thus, it is difficult to obtain a medicine amount effective in preventing restenosis . On the other hand, a ratio of more than 0.40 is also unfavorable, because it leads to more frequent cracking and exfoliation of the external layer associated with stent expansion and also to insufficient slow-drug-delivery property. The stent according to the present invention is a stent for placement in body having a medicine/polymer weight ratio in the internal layer of 0.50 or more and 1.60 or less and a medicine/polymer weight ratio in the external layer of 0.10 or more and 0.40 or less.

EXAMPLES

(Example 1)

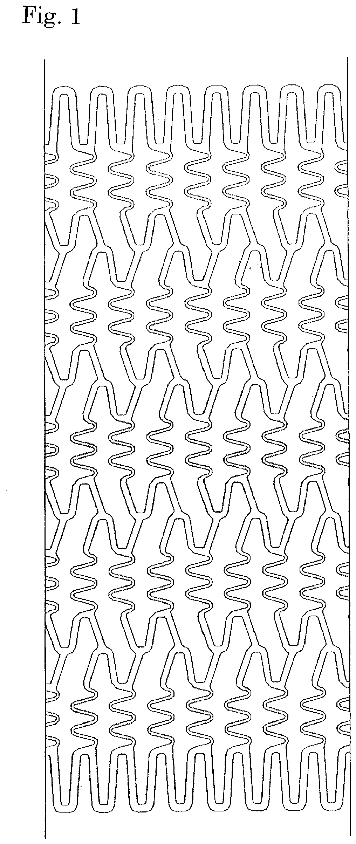

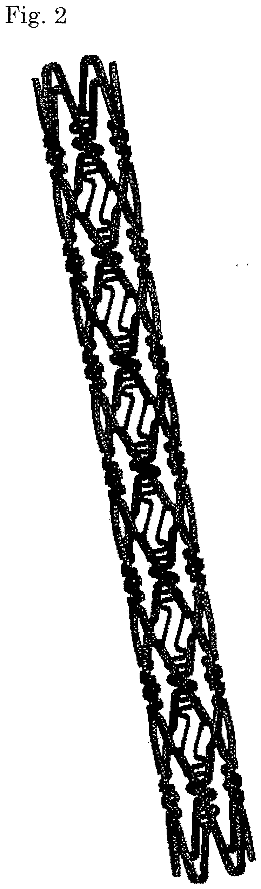

A stent main body was prepared by cutting a stainless steel tube (SUS316L) having an internal diameter of 1.50 mm and an external diameter of 1. 80 mm into the stent shape by laser cutting and polishing it electrolytically, similarly to the method normally practiced by those who are skilled in the art. Figure 1 is a development view of the stent, and Figure 2 is a schematic view thereof. The length of the stent was set to 13 mm, the thickness to 120 $\mu$m, and the nominal diameter after expansion to 3.5 mm. The stent is a so-called balloon expandable stent that is inflated and placed by using a balloon catheter having a balloon in the region of the catheter close to the distal end. The balloon expandable stent, which is placed in the balloon region of the balloon catheter as it is contracted, is delivered to a desired site and inflated and placed there by expansion of the balloon.

A lactic acid-glycolic acid copolymer (product number: 85DG065, manufactured by Absorbable Polymers International, lactic acid/glycolic acid: 85/15, weight-average molecular weight 85, 000) as the polymer and a medicine tacrolimus (Fujisawa Pharmaceutical Co., Ltd.) were dissolved in chloroform (Wako Pure Chemical Industries Ltd.), to give a solution containing the medicine and the polymer respectively at concentrations of 0.50 wt % and 0.50 wt %. A stainless steel wire having a diameter of 100 $\mu$m was connected to one end of the stent, and the other end was connected to a stainless steel having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed by using a spray gun having a nozzle diameter of 0.3 mm on the stent, allowing deposition of the solution. The distance between the nozzle of spray gun and the stent was 75 mm, and the air pressure during spraying was 0.15 MPa. The stent was dried after spraying under vacuum at room temperature for 1 hour. An internal layer (medicine/polymer weight ratio: 1.00) having a polymer weight per stent of 100 pg and a medicine weight of 100 pg was formed, while the spraying period was adjusted.

A lactic acid-glycolic acid copolymer (product number: 85DGO65, manufactured by Absorbable Polymers International, lactic acid/glycolic acid: 85/15, weight-average molecular weight 85, 000) as the polymer and a medicine tacrolimus (Fujisawa Pharmaceutical Co., Ltd.) were dissolved in chloroform (Wako Pure Chemical Industries Ltd.), to give a solution containing the medicine and the polymer respectively at concentrations of 0.13 wt % and 0.50 wt %. A stainless steel wire having a diameter of 100 $\mu$m was connected to one end of a stent having the formed internal layer, and the other end was connected to a stainless steel rod having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed by using a spray gun having a nozzle diameter of 0.3 mm on the stent, allowing deposition of the solution. The distance between the nozzle of spray gun and the stent was 75 mm, and the air pressure during spraying was 0.15 MPa. The stent was dried after spraying under vacuum at room temperature for 1 hour. An external layer (medicine/polymer weight ratio: 0.26) having a polymer weight per stent of 192 $\mu$g and a medicine weight of 50 $\mu$g was formed, while the spraying period was adjusted.

The total weight of the polymer both in the internal and external layer per stent obtained was 292 $\mu$g, and the weight of the medicine was 150 $\mu$g (medicine/polymer weight ratio: 0.51) .

(Example 2)

A stent was prepared in a similar manner to Example 1, except that a solution at a medicine concentration/polymer concentration rate of 0.75 wt %/0.50 wt % was prepared and an internal layer (medicine/polymer weight ratio: 1.52) having a polymer weight per stent of 66 $\mu$g and a medicine weight of 100 $\mu$g was formed.

The total weight of the polymer both in the internal and external layer per stent obtained was 258 $\mu$g, and the weight of the medicine was 150 $\mu$g (medicine/polymer weight ratio : 0.58).

(Example 3)

A stent was prepared in a similar manner to Example 1, except that a solution at a medicine concentration/polymer concentration rate of 0.05 wt %/0.50 wt % was prepared and an external layer (medicine/polymer weight ratio: 0.10) having a polymer weight per stent of 500 $\mu$g and a medicine weight of 50 pg was formed.

The total weight of the polymer both in the internal and external layer per stent obtained was 600 $\mu$g, and the weight of the medicine was 150 pg (medicine/polymer weight ratio: 0.25).

(Example 4)

A stent was prepared in a similar manner to Example 1, except that a solution at a medicine concentration/polymer concentration rate of 0.75wt %/0.50wt % was prepared, an internal layer (medicine/polymer weight ratio: 1.52) having a polymer weight per stent of 66 pg and a medicine weight of 100 $\mu$g was formed, a solution at a medicine/polymer rate of 0.20 wt %/0.50 wt % was prepared, and an external layer (medicine/polymer weight ratio: 0.40) having a polymer weight per stent of 125 pg and a medicine weight of 50 $\mu$g was formed.

The total weight of the polymer both in the internal and external layer per stent obtained was 191 $\mu$g, and the weight of the medicine was 150 $\mu$g (medicine/polymer weight ratio : 0.79).

(Example 5)

A stent was prepared in a similar manner to Example 1, except that a solution at a medicine concentration/polymer concentration rate of 0.25 wt %/0.50 wt % was prepared and an internal layer (medicine/polymer weight ratio: 0.50) having a polymer weight per stent of 200 pg and a medicine weight of 100 pg was formed.

The total weight of the polymer both in the internal and external layer per stent obtained was 392 $\mu$g, and the weight of the medicine was 150 $\mu$g (medicine/polymer weight ratio: 0.38).

(Comparative Example 1)

A stent main body was prepared by cutting a stainless steel tube (SUS316L) having an internal diameter of 1.50 mm and an external diameter of 1.80 mm into the stent shape by laser cutting and polishing it electrolytically, similarly to the method normally practiced by those who are skilled in the art. Figure 1 is a development view of the stent, and Figure 2 is a schematic view thereof. The length of the stent was set to 13 mm, the thickness to 120 $\mu$m, and the nominal diameter after expansion to 3.5 mm. The stent is a so-called balloon expandable stent that is inflated and placed by using a balloon catheter having a balloon in the region of the catheter close to the distal end. The balloon expandable stent, which is placed in the balloon region of the balloon catheter as it is contracted, is delivered to a desired site and inflated and placed there by expansion of the balloon.

A lactic acid-glycolic acid copolymer (product number: 85DG065, manufactured by Absorbable Polymers International, lactic acid/glycolic acid: 85/15, weight-average molecular weight 85,000) as the polymer and a medicine tacrolimus (Fujisawa Pharmaceutical Co., Ltd.) were dissolved in chloroform (Wako Pure Chemical Industries Ltd.), to give a solution containing the medicine and the polymer respectively at concentrations of 0.50 wt % and 0.50 wt %. A stainless steel wire having a diameter of 100 $\mu$m was connected to one end of the stent, and the other end was connected to a stainless steel having a diameter of 2 mm. The stent was held in the direction perpendicular to the length direction, by connecting the stent-unconnected sided terminal of the stainless steel rod to a motor. The stent was rotated with the motor at a frequency of 100 rpm, and the solution prepared was sprayed by using a spray gun having a nozzle diameter of 0.3 mm on the stent, allowing deposition of the solution. The distance between the nozzle of spray gun and the stent was 75 mm, and the air pressure during spraying was 0.15 MPa. The stent was dried after spraying under vacuum at room temperature for 1 hour. A coating layer (medicine/polymer weight ratio: 1.00) having a polymer weight per stent of 150 pg and a medicine weight of 150 $\mu$g was formed, while the spraying period was adjusted.

(Comparative Example 2)

A stent was prepared in a similar manner to Comparative Example 1, except that a solution at a medicine concentration/polymer concentration rate of 0.13 wt % /0.50 wt % was prepared and a coating layer (medicine/polymer weight ratio: 0.26) having a polymer weight per stent of 580 pg and a medicine weight of 150 $\mu$g was formed.

(In-vitro evaluation experiment 1)

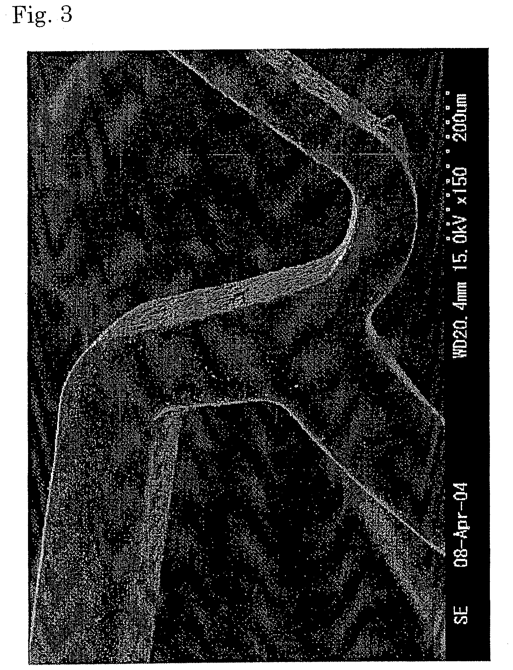

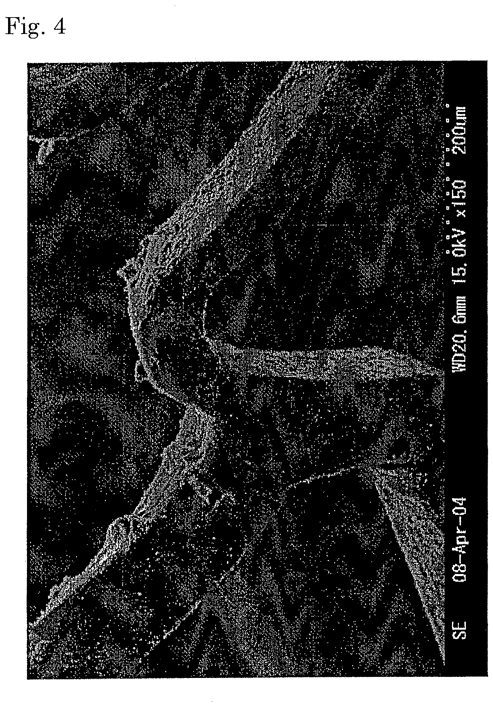

A PTCA balloon catheter containing a balloon of 3.5$\times$15 mm in dimension was prepared, and the stent described above was mounted on the balloon region. The balloon was inflated in air at room temperature at 8 atm (810kPa), allowing expansion of the stent. The balloon was deflated after 1 minute and separated from the stent. The expanded stent was fixed on the test-piece stage of an electron microscope and vapor-deposited with a Pt-Pd alloy, and the surface thereof was observed under a scanning electron microscope (S-3000N, manufactured by Hitachi High-Technologies Corp.). The frequency of cracking and exfoliation on the coated film was evaluated qualitatively, and the results are summarized in Table 1. Figure 3 shows an SEM observation image of the sample in Example 1 and Figure 4 shows a SEM observation image of the sample in Comparative Example 1, as typical examples of the cracking and exfoliation on the coated film.

(In-vitro evaluation experiment 2)

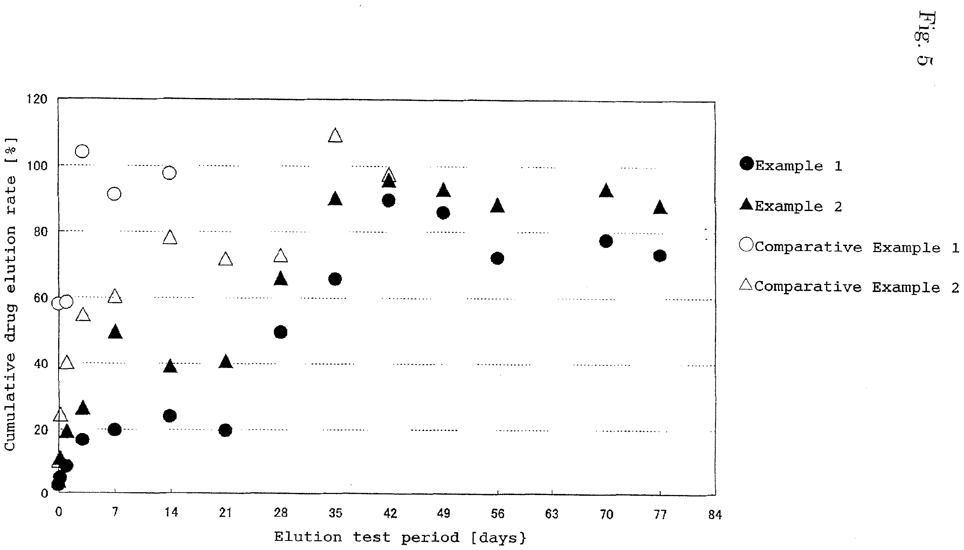

Elution test of the medicines used in Examples 1 and 2 and Comparative Examples 1 and 2 was performed. Each stent was immersed in 100 mL of an acidic phosphate buffer (pH 3.4, NaCl: 6.1 g/L, $NaH_2PO_4$-$2H_2O$: 7.1 g/L, $H_3PO_4$: 263 $\mu$L/L) containing 35% methanol and stirred in a water bath at 37° C. A 1-mL sample was collected at a certain interval, and the buffer was replenished with the equal amount of fresh buffer solution, to keep the total amount of the test solution always at 100 mL. The concentration of tacrolimus in the sample solution was determined quantitatively by HPLC (Alliance, manufactured by Nihon Waters K.K.), and calculated according to the following Formula. Results (data) are summarized in Table 2 and the graph in Figure 5.

```
Elution rate (%) =

Tacrolimus concentration in sample solution (wt %) /

Tacrolimus concentration when all stent tacrolimus is eluted

  into buffer solution (wt %) ×100
```

[Table 1]

| | Internal layer | | | External layer | | | Total | | | Cracking | Exfoliation |
|---|---|---|---|---|---|---|---|---|---|---|---|
| | Medicine amount [μg] | Polymer amount [μg] | Medicine/polymer weight ratio | Medicine amount [μg] | Polymer amount [μg] | Medicine/polymer weight ratio | Medicine amount [μg] | Polymer amount [μg] | Medicine/polymer weight ratio | | |
| Example 1 | 100 | 100 | 1.00 | 50 | 192 | 0.26 | 150 | 292 | 0.51 | ○ | ○ |
| Example 2 | 100 | 66 | 1.52 | 50 | 192 | 0.26 | 150 | 258 | 0.58 | ○ | ○ |
| Example 3 | 100 | 100 | 1.00 | 50 | 500 | 0.10 | 150 | 600 | 0.25 | ○ | ○ |
| Example 4 | 100 | 66 | 1.52 | 50 | 125 | 0.40 | 150 | 191 | 0.79 | ○ | ○ |
| Example 5 | 100 | 200 | 0.50 | 50 | 192 | 0.26 | 150 | 392 | 0.38 | ○ | ○ |
| Comparative Example 1 | 150 | 150 | 1.00 | - | - | - | 150 | 150 | 1.00 | × | × |
| Comparative Example 2 | 150 | 580 | 0.26 | - | - | - | 150 | 580 | 0.26 | ○ | ○ |

As shown in Table 1, the stents according to the invention obtained in Examples 1 to 5 showed no cracking or exfoliation of the coating layer associated with stent expansion and had favorable surface properties. On the other hand, the stent obtained in Comparative Example 1 showed the cracking and exfoliation of the coating layer associated with stent expansion. In addition, the stent obtained in Comparative Example 2 showed slight cracking and was inferior to the stent obtained in Example 3 that has almost the same medicine/polymer weight ratio in the entire stent.

[Table 2]

| Elution test period [day] | Elution rate[%] | | | |
|---|---|---|---|---|
| | Example 1 | Example 2 | Comparative Example 1 | Comparative Example 2 |
| 0.04 | 2.5 | 3.7 | 57.6 | 10.0 |
| 0.25 | 4.7 | 10.8 | - | 24.3 |
| 1 | 8.3 | 19.3 | 58.3 | 40.2 |
| 3 | 16.5 | 26.6 | 103.7 | 54.5 |
| 7 | 19.7 | 49.6 | 91.1 | 60.3 |
| 14 | 23.9 | 39.1 | 97.5 | 78.5 |
| 21 | 19.5 | 40.7 | - | 71.8 |
| 28 | 49.3 | 65.7 | - | 72.8 |
| 35 | 65.5 | 90.3 | - | 109.5 |
| 42 | 89.5 | 95.8 | - | 97.4 |
| 49 | 85.9 | 93.0 | - | - |
| 56 | 72.0 | 88.5 | - | - |
| 70 | 77.5 | 93.3 | - | - |
| 77 | 73.1 | 88.3 | - | - |

As shown in Figure 5, the stent of Comparative Example 2 (entire medicine/polymer weight ratio of stent: 0.26) had a medicine/polymer weight ratio lower than that of the stent of Comparative Example 1 (entire medicine/polymer weight ratio of stent: 1.00), and thus, the medicine is eluted therefrom in a controlled manner. The stents according to the invention obtained in Example 1 (entire medicine/polymer weight ratio of the stent: 0.51) and Example 2 (entire medicine/polymer weight ratio of the stent: 0.58) released the medicine in more distinctively controlled way than those obtained in Comparative Example 1 and 2, which is probably because of the presence of the internal and external layers. In addition, the stents obtained in Examples 1 and 2 had a relatively high entire medicine/polymer weight ratio of the stent and thus had a significantly larger medicine retention rate.
As described above, in the Examples above according to the invention, it is possible to increase the amount of the medicine retained on the stent and allow release of the medicine in a controlled manner without cracking and exfoliation of the coating layer associated with stent expansion.

**Claims**

1. A tube-shaped stent expandable toward outside in the radial direction of the tube expanding, **characterized by** including a stent main body containing a material non-degradable in the body as its base stent material, a coating layer containing a medicine and a polymer formed on at least part of the stent main body surface, wherein the coating layer consists of an internal layer and an external layer, the medicine/polymer weight ratio by weight in the internal layer is higher than that in the external layer, and the medicine is contained in an effective amount in the external layer wherein the medicine\polymer weight ratio in the internal layer is 0.50 or more and 1.60 or less and the medicine\polymer weight ratio in the external layes is 0-10 or more and 0-40 or less.

2. The stent for placement in body according to Claim 1, wherein the coating layer covers the almost entire surface of the external, internal, and side faces of the stent main body.

3. The stent for placement in body according to Claim 1, wherein the polymer is a biodegradable polymer.

4. The stent for placement in body according to Claim 3, wherein the biodegradable polymer is at least one polymer selected from polylactic acid, polyglycolic acid, and lactic acid-glycolic acid copolymers.

5. The stent for placement in body according to Claim 4, wherein the biodegradation period of the polymer for the internal layer is almost same as that of the polymer for the external layer.

6. The stent for placement in body according to Claim 4, wherein the biodegradation period of the polymer for the internal layer is longer than that of the polymer for the external layer.

7. The stent for placement in body according to any one of Claims 1 to 6, wherein the medicine contained in the internal layer is the same as that contained in the external layer.

8. The stent for placement in body according to Claim 7, wherein the medicine is an immunosuppressive agent.

9. The stent for placement in body according to Claim 8, wherein the immunosuppressive agent is at least one compound selected from tacrolimus (FK506), cyclosporine, sirolimus (rapamycin), azathioprine, and mycophenolate mofetil.

10. The stent for placement in body according to Claim 9, wherein the immunosuppressive agent is tacrolimus (FK506).

11. The stent for placement in body according to any one of Claims 1 to 6, wherein the medicine contained in the internal layer is different from that contained in the external layer.

12. The stent for placement in body according to Claim 11, wherein the medicine contained in the internal layer is an immunosuppressive agent and the medicine contained in the external layer is an anti-inflammatory agent.

13. The stent for placement in body according to Claim 12, wherein: the immunosuppressive agent is at least one compound selected from tacrolimus (FK506), cyclosporine, sirolimus (rapamycin), azathioprine, mycophenolate mofetil and the analogs thereof; and the antiinflammatory agent is at least one compound selected from dexamethasone, hydroxycortisone, cortisone, desoxycorticosterone, fludrocortisone, betamethasone, prednisolone, prednisone, methyl prednisolone, paramethasone, triamcinolone, flumethasone, fluocinolone, fluocinonide, fluprednisolone, halcinonide, flurandrenolide, meprednisone, medrysone, cortisol, 6a-methyl prednisolone, triamcinolone, betamethasone, salicylic acid derivative, diclofenac, naproxen, sulindac, and indomethacin.

14. The stent for placement in body according to Claim 13, wherein the immunosuppressive agent is tacrolimus (FK506) and the anti-inflammatory agent is dexamethasone.

15. The stent for placement in body according to Claim 1, wherein the medicine/polymer weight ratio in the internal layer is 0.50 or more and 1.60 or less.

16. The stent for placement in body according to Claim 1, wherein the medicine/polymer weight ratio in the external layer is 0.10 or more and 0.40 or less.

**Patentansprüche**

1. Röhrenförmiger Stent, der nach außen in radialer Richtung zu der Rohrerstreckung ausdehnbar ist, **dadurch gekennzeichnet, dass** er einen Stentgrundkörper aufweist, der als sein Stentbasismaterial ein im Körper sich nicht zersetzendes Material aufweist, und eine ein Arzneimittel und ein Polymer enthaltende Deckschicht, die auf zumindest einem Teil der Stentgrundkörperfläche gebildet ist, wobei die Deckschicht aus einer inneren Schicht und ein äußeren Schicht besteht, wobei das Arzneimittel-Polymer-Gewichtsverhältnis in der inneren Schicht größer ist als in der äußeren Schicht, und das Arzneimittel in einer wirksamen Menge in der äußeren Schicht enthalten ist, wobei das Arzneimittel-Polymer-Gewichtsverhältnis in der inneren Schicht 0-50 oder mehr und 1-60 oder weniger beträgt und das Arzneimittel-Polymer-Gewichtsverhältnis in der äußeren Schicht 0-10 oder mehr und 1-40 oder weniger beträgt.

2. Stent zur Anordnung im Körper gemäß Anspruch 1, wobei die Deckschicht nahezu die gesamte Oberfläche der äußeren, inneren und seitlichen Flächen des Stentgrundkörpers abdeckt.

3. Stent zur Anordnung im Körper gemäß Anspruch 1, wobei das Polymer ein biologisch abbaubares Polymer ist.

4. Stent zur Anordnung im Körper gemäß Anspruch 3, wobei das biologisch abbaubare Polymer zumindest ein Polymer ist, das ausgewählt ist aus Polymilchsäure, Polyglykolidsäure, und Milchsäure-Glykolsäure-Copolymeren.

5. Stent zur Anordnung im Körper gemäß Anspruch 4, wobei der biologische Abbauzeitraum des Polymers der inneren Schicht nahezu der gleiche des Polymers der äußeren Schicht ist.

6. Stent zur Anordnung im Körper gemäß Anspruch 4, wobei der biologische Abbauzeitraum des Polymers der inneren Schicht länger als derjenige des Polymers der äußeren Schicht ist.

7. Stent zur Anordnung im Körper gemäß irgendeinem der Ansprüche 1 bis 6, wobei das in der inneren Schicht enthaltene Arzneimittel dasselbe ist wie das in der äußeren Schicht enthaltene.

8. Stent zur Anordnung im Körper gemäß Anspruch 7, wobei das Arzneimittel ein immunosuppressives Medikament ist.

9. Stent zur Anordnung im Körper gemäß Anspruch 8, wobei das immunosuppressive Medikament zumindest eine Verbindung ist, die ausgewählt ist aus Tacrolimus (FK506), Ciclosporin, Sirolimus (Rapamycin) Azathioprin und Mycophenolat-Mofetil.

10. Stent zur Anordnung im Körper gemäß Anspruch 9, wobei das immunosuppressive Medikament Tacrolimus (FK506) ist.

11. Stent zur Anordnung im Körper gemäß irgendeinem der Ansprüche 1 bis 6, wobei das in der inneren Schicht enthaltene Arzneimittel sich von demjenigen, das in der äußeren Schicht enthalten ist, unterscheidet.

12. Stent zur Anordnung im Körper gemäß Anspruch 11, wobei das in der inneren Schicht enthaltene Arzneimittel ein immunosuppressives Medikament und das in der äußeren Schicht enthaltene Arzneimittel ein entzündungshemmendes Mittel ist.

13. Stent zur Anordnung im Körper gemäß Anspruch 12, wobei: das immunosuppressive Medikament wenigstens eine Verbindung ist, die ausgewählt ist aus Tacrolimus (FK506), Ciclosporin, Sirolimus (Rapamycin) Azathioprin, Mycophenolat-Mofetil und deren Nachbildungen; und das entzündungshemmende Mittel wenigstens eine Verbindung ist, die ausgewählt ist aus Dexamethason, Hydroxykortison, Kortison, Desoxycorticosteron, Fludrokortison, Betamethason, Prednisolon, Prednison, Methylprednisolon, Paramethason, Triamcinolon, Flumethason, Fluocinolon, Fluocinoid, Fluprednisolon, Halcinoid, Flurandrenolid, Meprednison, Medryson, Cortisol, 6a-Methyl-Prednisolon, Triamcinolon, Betamethason, Salicylsäurederivat, Diclofenac, Naproxen, Sulindac und Indomethacin.

14. Stent zur Anordnung im Körper gemäß Anspruch 12, wobei das immunosuppressive Medikament Tacrolimus (FK506) und das entzündungshemmende Mittel Dexamethason ist.

15. Stent zur Anordnung im Körper gemäß Anspruch 1, wobei das Arzneimittel-Polymer-Gewichtsverhältnis in der inneren Schicht 0,50 oder mehr und 1,60 oder weniger beträgt.

16. Stent zur Anordnung im Körper gemäß Anspruch 1, wobei das Arzneimittel-Polymer-Gewichtsverhältnis in der äußeren Schicht 0,10 oder mehr und 0,40 oder weniger beträgt.

**Revendications**

1. Endoprothèse tubulaire expansible vers l'extérieur dans la direction radiale de l'extension du tube, **caractérisée en ce qu'**elle comprend un corps principal d'endoprothèse contenant un matériau non-dégradable dans le corps comme le matériau de base d'endoprothèse de celle-ci et une couche couvrante contenant un médicament et un polymère formée sur au moins une partie de la surface du corps principal d'endoprothèse, la couche couvrante étant composée d'une couche intérieure et d'une couche extérieure, le rapport de poids médicament/polymère dans la couche intérieure étant supérieur à celui dans la couche extérieure, et une quantité efficace du médicament étant contenue dans la couche extérieure, le rapport de poids médicament/polymère dans la couche intérieure étant 0 à 50 ou plus et 1 à 60 ou moins et le rapport de poids médicament/polymère dans la couche extérieure étant 0 à 10 ou plus et 0 à 40 ou moins.

2. Endoprothèse destinée à être disposée dans un corps selon la revendication 1, dans laquelle la couche couvrante couvre presque la surface entière des surfaces extérieures, intérieures et latérales du corps principal d'endoprothèse.

3. Endoprothèse destinée à être disposée dans un corps selon la revendication 1, dans laquelle le polymère est un polymère bio-dégradable.

4. Endoprothèse destinée à être disposée dans un corps selon la revendication 3, dans laquelle le polymère bio-dégradable est au moins un polymère sélectionné parmi l'acide polylactique, l'acide polyglycolique et des copolymères de l'acide lactique/l'acide glycolique.

5. Endoprothèse destinée à être disposée dans un corps selon la revendication 4, dans laquelle la période de bio-dégradation du polymère pour la couche intérieure est presque la même que celle du polymère pour la couche extérieure.

6. Endoprothèse destinée à être disposée dans un corps selon la revendication 4, dans laquelle la période de bio-dégradation du polymère pour la couche intérieure est plus longue que celle du polymère pour la couche extérieure.

7. Endoprothèse destinée à être disposée dans un corps selon l'une quelconque des revendications 1 à 6, dans laquelle le médicament contenu dans la couche intérieure est le même que celui contenu dans la couche extérieure.

8. Endoprothèse destinée à être disposée dans un corps selon la revendication 7, dans laquelle le médicament est un agent immunosuppressif.

9. Endoprothèse destinée à être disposée dans un corps selon la revendication 8, dans laquelle l'agent immunosuppressif est au moins un composé sélectionné parmi le tacrolimus (FK506), la ciclosporine, le sirolimus (la rapamycin), l'azathioprine et le Mycophénolate-Mofetil.

10. Endoprothèse destinée à être disposée dans un corps selon la revendication 9, dans laquelle l'agent immunosuppressif est le tacrolimus (FK506).

11. Endoprothèse destinée à être disposée dans un corps selon l'une quelconque des revendications 1 à 6, dans laquelle le médicament contenu dans la couche intérieure est différent de celui contenu dans la couche extérieure.

12. Endoprothèse destinée à être disposée dans un corps selon la revendication 11, dans laquelle le médicament contenu dans la couche intérieure est un agent immunosuppressif et le médicament contenu dans la couche extérieure est un agent anti-inflammatoire.

13. Endoprothèse destinée à être disposée dans un corps selon la revendication 12, dans laquelle l'agent immunosuppressif est au moins un composé sélectionné parmi le tacrolimus (FK506), la ciclosporine, le sirolimus (la rapamycin), l'azathioprine, le Mycophénolate-Mofetil et des analogues de ceux-ci ; et l'agent anti-inflammatoire est au moins un composé sélectionné parmi la dexaméthasone, l'hydroxycortisone, la cortisone, la desoxycorticostérone, la fludrocortisone, la bétaméthasone, la prednisolone, la prednisone, la méthylprednisolone, la paraméthasone, la triamcinolone, la fluméthasone, la fluocinolone, le fluocinonide, la fluprednisolone, la halcinoïde, le flurandrénolide, la méprednisone, la médrysone, le cortisol, la 6a-méthylprednisolone, la triamcinolone, la bétaméthasone, un dérivé de l'acide salicylique, le diclofénac, le naproxène, le sulindac et l'indométhacine.

14. Endoprothèse destinée à être disposée dans un corps selon la revendication 13, dans laquelle l'agent immunosuppressif est le tacrolimus (FK506) et l'agent immunosuppressif est la déxaméthasone.

15. Endoprothèse destinée à être disposée dans un corps selon la revendication 1, dans laquelle le rapport de poids médicament/polymère dans la couche intérieure est 0,50 ou plus et 1,60 ou moins.

16. Endoprothèse destinée à être disposée dans un corps selon la revendication 1, dans laquelle le rapport de poids médicament/polymère dans la couche extérieure est 0,10 ou plus et 0,40 ou moins.

Fig. 1

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5824048 A
- JP 5502179 A
- JP 6009390 A
- JP 9503488 A
- WO 02065947 A
- EP 1254674 A
- JP 61148181 A
- JP 2002531183 A
- JP 2004000754 A

**Non-patent literature cited in the description**

- **PAUL J. MOHACSI MD et al.** *The Journal of Heart and Lung Transplantation,* May 1997, vol. 16 (5), 484-491